(19) Europäisches Patentamt
European Patent Office
Office européen des brevets

(11) Veröffentlichungsnummer: **0 000 353**
**A2**

(12) # EUROPÄISCHE PATENTANMELDUNG

(21) Anmeldenummer: **78100272.0**

(22) Anmeldetag: **29.06.78**

(51) Int. Cl.[2]: **C07D513/04,** C07D277/18, A61K31/425, A61K31/54 // C07D233/84, C07D213/53, C07D213/50, C07D401/04 , (C07D513/04, 277/00, 235/00, 513/04, 279/00, 235/00, 401/04, 233/84, 213/38)

(30) Priorität: **07.07.77 LU 77703**

(43) Veröffentlichungstag der Anmeldung: **24.01.79 Patentblatt 79/2**

(84) Benannte Vertragsstaaten: **BE CH DE FR GB NL SE**

(71) Anmelder: **CIBA-GEIGY AG, Patentabteilung Postfach, CH-4002 Basel (CH)**

(72) Erfinder: **Göschke, Richard, Dr., Felix-Häglistrasse 21, CH-4103 Bottmingen (CH)**
**Ferrini, Pier Giorgio, Dr., Im Rehwechsel 22, CH-4102 Binningen (CH)**

(74) Vertreter: **Zumstein sen., Fritz, Dr. et al, Bräuhausstrasse 4, D-8000 München 2 (DE)**

(54) **Bicyclische Thiadiazaverbindungen, Verfahren und Zwischenprodukte bei deren Herstellung, und Arzneimittel welche diese Verbindungen oder Zwischenprodukte enthalten.**

(57) Bicyclische Thia-diaza-verbindungen, insbesondere 1,3-Diaza-cyclopent-2-eno[2,1-b]-(1-thia-3-aza-cycloalkane) der allgemeinen Formel

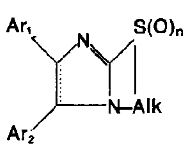

(I)

deren 1,3-Diaza-cyclopent-2-en-ring eine weitere Doppelbindung aufweisen kann, Alk Niederalkylen darstellt, welches das Thia- vom Aza-atom durch 2–4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschieden ist, wenn Alk Äthylen und der 1,3-Diaza-cyclopent-2-en-ring einen Imidazolring darstellt, und deren Salze, sowie Verfahren zu ihrer Herstellung.

Die neuen Verbindungen I und Zwischenverbindungen der Formel Va bzw. Vb

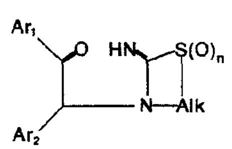

(Va)

bzw.

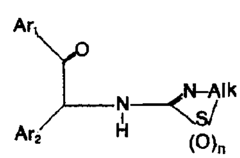

(Vb)

besitzen wertvolle pharmakologische Eigenschaften.

EP 0 000 353 A2

4-11226/+

# Neue bicyclische Thiadiaza-verbindungen und Verfahren zu deren Herstellung

Die Erfindung betrifft neue bicyclische Thiadiaza-verbindungen, insbesondere 1,3-Diaza-cyclopent-2-eno [2,1-b](1-thia-3-aza-cycloalkane) der allgemeinen Formel (I)

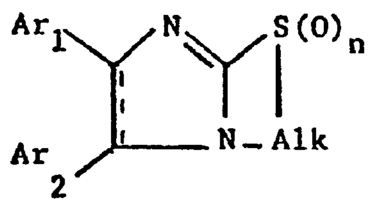

(I)

deren 1,3-Diaza-cyclopent-2-en-ring eine weitere Doppelbindung aufweisen kann, Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschieden ist, wenn Alk Aethylen und der 1,3-Diaza-cyclopent-2-en-ring einen Imidazolring darstellt, und deren Salze, sowie Verfahren zu ihrer Herstellung, ferner pharmazeutische Präparate enthaltend diese Verbindungen und ihre Verwendung, vorzugsweise in Form von pharmazeutischen Präparaten.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Niederalkylen Alk ist vorzugsweise unverzweigtes, aber auch verzweigtes Niederalkylen mit 2-4 Kohlenstoffatomen in der Kette zwischen dem Schwefel- und dem Stickstoffatom.

Pyridyl ist ein 2-, 3- oder 4-Pyridyl und Thienyl ein 3- oder insbesondere 2-Thienyl.

Substituiertes Phenyl, Pyridyl oder Thienyl ist z.B. einfach, zweifach oder auch mehrfach substituiert. Substituenten, insbesondere am Phenylrest sind u.a. Niederalkyl, Niederalkoxy, Halogen, Trifluormethyl, Niederalkylsulfonyl oder Nitro. Substituenten am Pyridyl- oder Thienyl-rest sind vorzugsweise Niederalkyl, Halogen oder Trifluormethyl.

Vorstehend wie nachfolgend können die Allgemeinbegriffe folgende Bedeutung haben:

Niederalkyl ist z.B. Methyl, Aethyl, n-Propyl, Isopropyl, n-Butyl, Isobutyl, sec.-Butyl oder tert.-Butyl, ferner n-Pentyl, n-Hexyl, Isohexyl oder n-Heptyl.

Niederalkylen ist Aethylen, sowie 1,3-Propylen, 1,4-Butylen, kann aber auch 1,2-Propylen, 1,2- oder 2,3-Butylen, 1,3- oder 2,4-Pentylen oder 1,4-Pentylen sein.

Niederalkoxy ist z.B. Methoxy, Aethoxy, n-Propyloxy, Isopropyloxy, n-Butyloxy oder tert.-Butyloxy.

Halogen ist solches mit der Atomnummer bis und mit 35 und steht für Fluor oder Brom, vorzugsweise für Chlor.

Niederalkylsulfonyl steht z.B. für Methylsulfonyl, Aethylsulfonyl oder n-Propylsulfonyl.

Die erfindungsgemässen Verbindungen besitzen wertvolle pharmakologische Eigenschaften, insbesondere entzündungshemmende und antirheumatische Wirkungen, wie sich in Tierversuchen zeigen lässt. z.B. im Kaolin-Pfotenoedem-Test (Helv. Physiol. Acta 25 (1967) 156) an der Ratte bei einer peroral gegebenen Dosis ab etwa 10 mg/kg oder im Terpentin-Pleuritis-Test [Helv. Physiol. Acta 26 (1969) 287] an der Ratte peroral gegeben bei einer Dosis von 30 bis 100 mg/kg zeigen sie eine antiinflammatorische bzw. anti-exsudative Wirkung. Insbesondere die ungesättigten Verbindungen zeigen auch im Adjuvans-Arthritis-Test [Pharmacology 2 (1969) 288] an der Ratte bei einer peroralen Dosis von 10-30 mg/kg eine ausgezeichnete Wirkung.

Die neuen Verbindungen sind auch analgetisch wirksam, wie sich im Phenyl-p-benzochinon-Test an der Maus (Proc. Soc. Exp. Biol. 95 (1957) 729) bei Dosen von 30 bis 100 mg/kg, peroral gegeben, zeigen lässt.

Ferner ist die Hemmwirkung der neuen Präparate auf die Prostaglandin-Synthetase in vitro [Prostaglandins, 7 (1974) 123] in Konzentrationen von 0.05-20 µg/ml zu nennen. Ausserdem zeigen sie einen wertvollen antithrombotischen Effekt, nämlich einen Schutz vor tödlicher Lungenembolie am Kaninchen [Pharmacology 14 (1976) 522] in peroralen Dosen von 0.03-3 mg/kg.

Die Tetrahydro-verbindungen zeigen zusätzlich dazu im Pertussisoedem-Test (Agents and Actions, vol. 6, 613, 1976) bei 5-50 mg/kg/Ratte eine verstärkende Wirkung.

Die neuen Verbindungen können desshalb als Antiphlogistika z.B. zur Behandlung von rheumatischen, arthritischen und anderen, mit Entzündungen verbundenen

Erkrankungen insbesondere, rheumatischer Arthritis oder als Analgetika, z.B. zur Behandlung von Schmerzzuständen, verwendet werden.

Die Erfindung betrifft insbesondere Verbindungen der Formel I, worin $Ar_1$ und $Ar_2$ unabhängig voneinander einen gegebenenfalls durch Niederalkyl, Niederalkoxy, Halogen oder Trifluormethyl substituierten Phenylrest, einen Pyridyl-, wie einen 2-, 3- oder 4-Pyridyl-, oder Thienyl-, insbesondere einen 2-Thienylrest darstellen, Alk einen Niederalkylenrest, der das Schwefel- und Stickstoffatom über 2-3 Kohlenstoffatome miteinander verbindet, in erster Linie einen unverzweigten Niederalkylenrest und n besonders 0, ferner auch 1 oder 2 bedeuten, und deren Salze.

Die Erfindung betrifft in erster Linie Verbindungen der Formel II

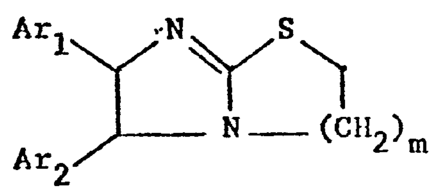

(II)

worin $Ar_1$ und $Ar_2$ unabhängig voneinander einen gegebenenfalls durch Niederalkoxy, wie Methoxy oder Halogen, insbesondere Chlor substituierten Phenylrest bedeuten und m in erster Line 1, ferner auch 2 ist, und ihre Salze.

Die Erfindung betrifft aber besonders auch Verbindungen der Formel III

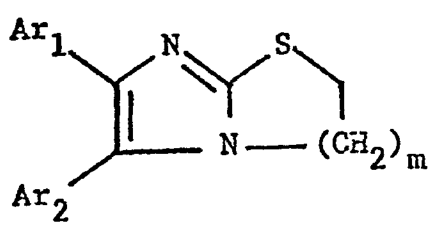

(III)

worin $Ar_1$ und $Ar_2$ unabhängig voneinander einen gegebenenfalls durch Niederalkoxy, wie Methoxy oder Halogen, insbesondere Chlor substituierten Phenylrest bedeuten und m in erster Linie 1, ferner auch 2 ist, und ihre Salze.

Insbesondere betrifft die Erfindung die neuen in den Beispielen beschriebenen Verbindungen.

Die neuen Verbindungen lassen sich nach an sich bekannten Methoden gewinnen.

So kann man z.B. Verbindungen der Formel IV

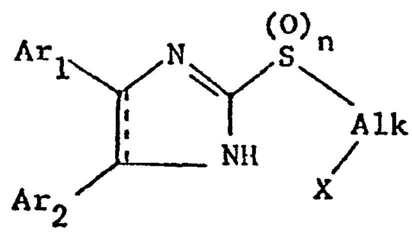

(IV)

worin X eine reaktionsfähige veresterte Hydroxygruppe darstellt, ringschliessen, und, wenn erwünscht, in gegebenenfalls erhaltenen Verbindungen, worin n 0 ist, das Thia - atom zur Sulfinyl- oder Sulfonylgruppe oxidieren, und/ oder, wenn erwünscht, erhaltene freie Verbindungen in ihre Salze überführen oder erhaltene Salze in die freien Verbindungen umwandeln und/oder ein verfahrensmässig erhaltenes Isomerengemisch in die einzelnen Isomeren trennen.

Reaktionsfähig verestertes Hydroxy ist insbesondere eine mit einer starken anorganischen Säure, z.B. Halogenwasserstoff, insbesondere Chlorwasserstoff, oder Schwefelsäure, oder mit einer starken organischen Säure, wie mit einer Niederalkansulfonsäure, z.B. Methan- oder Aethansulfonsäure oder einer gegebenenfalls durch Niederalkyl, Niederalkoxy oder Halogen substituierten Benzolsulfonsäure, z.B. p-Toluolsulfonsäure oder p-Brombenzolsulfonsäure veresterte Hydroxygruppe.

Der Ringsschluss wird vorzugsweise unter Säureabspaltenden Bedingungen durchgeführt. Dabei arbeitet man in erster Linie in einem niedersiedenden Lösungsmittel, wie Dimethylformamid oder Aceton, einem Alkohol, z.B. Methanol oder Aethanol, wenn erwünscht, in Gegenwart einer Base, z.B. einer anorganischen Base, wie einem Alkali- oder Erdalkalihydrid, -hydroxyd oder -carbonat, in erster Linie Natriumhydrid, Natriumhydroxyd oder Natriumcarbonat, oder einer organischen Base, vorzugsweise einer Stickstoffbase, wie Triniederalkylamin, z.B. Trimethylamin, Triäthylamin, Dimethyl-isopropylamin, oder Pyridin.

Die Ausgangsstoffe lassen sich erhalten, wenn man eine ensprechende Diaza-2-mercapto-verbindung mit einem Dihydroxyalkylen, worin mindestens eine der beiden Hydroxygruppen reaktionsfähig verestert ist, miteinander umsetzt, gegebenenfalls die Mercaptogruppe zur Sulfinyl- oder Sulfonylgruppe oxidiert und anschliessend, falls notwendig, die zweite Hydroxygruppe reaktionsfähig verestert. Die reaktionsfähig veresterten Hydroxygruppen entsprechen den obgenannten wie auch die Bedingungen der Kondensation. Bei dieser Reaktion können, insbesondere wenn beide Hydroxylgruppen reaktionsfähig verestert sind, die Ausgangsstoffe der Formel IV in situ erhalten werden, welche in der gleichen Reaktion zum Ring geschlossen werden.

Die neuen Verbindungen der Formel I, die eine weitere Doppelbindung aufweisen, können auch erhalten werden, wenn man Verbindungen der Formel Va bzw. Vb

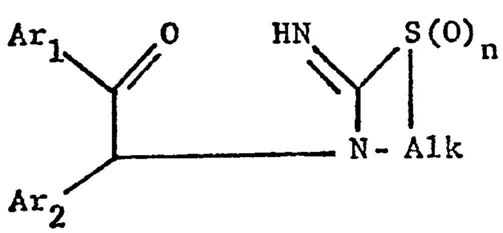

(Va)

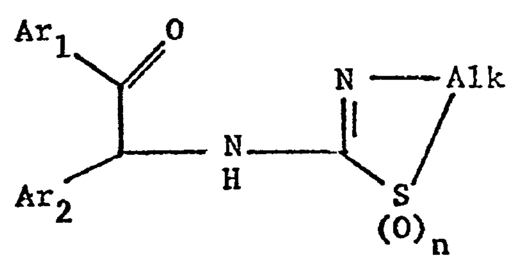

(Vb)

oder ihre Tautomeren ringschliesst, und wenn erwünscht, in gegebenenfalls erhaltenen Verbindungen, worin n 0 ist, das Thia-atom zur Sulfinyl- oder Sulfonylgruppe oxidiert, und/oder, wenn erwünscht, erhaltene freie Verbindungen in ihre Salze überführt oder erhaltene Salze in die freien Verbindungen umwandelt und/oder ein verfahrensgemäss erhaltenes Isomerengemisch in die einzelnen Isomeren auftrennt.

Der Ringschluss erfolgt unter wasserabspaltenden Bedingungen, wie durch Erwärmen, z.B. von etwa 50° bis etwa 150°, vorzugsweise in Gegenwart eines Lösungsmittels, wie Acetonitril oder einem Alkohol, z.B. Methanol oder Aethanol.

Die Ausgangsstoffe lassen sich erhalten, wenn man in an sich bekannter Weise eine Verbindung der Formel VI

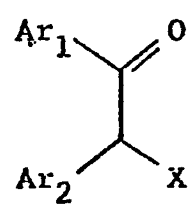

(VI)

mit einer Verbindung der Formel VII

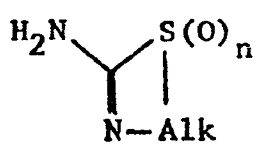

(VII)

oder ihren Tautomeren umsetzt.

Reaktionsfähig veresterte Hydroxygruppen X sind insbesondere die obgenannten.

Wird diese Umsetzung auch unter wasserabspaltenden Bedingungen vorgenommen, z.B. unter Erwärmen in einem Lösungsmittel wie Acetonitril oder einem Alkohol,

kann der Ausgangsstoff der Formel Va bzw. Vb in situ erhalten werden, der sich unter diesen Reaktionsbedingungen ringschliesst.

Die Oxydation des Thia-atoms zur Sulfinyl- oder Sulfonylgruppe lässt sich in an sich bekannter Weise z.B. mit Peroxyden, wie Wasserstoffperoxyd, oder Persäuren, z.B. einer gegebenenfalls durch Niederalkyl, Niederalkoxy, Halogen oder einer weiteren Carboxylgruppe substituierten Benzoepersäure, wie Benzoepersäure selbst oder Phthalmonopersäure, oder einer Alkanpercarbonsäure, wie Peressigsäure oder einem Perjodat, wie Natriumperjodat durchführen. Diese Reaktion wird meist bei tiefen Temperaturen in einem Lösungsmittel, wie Eisessig oder Aceton vorgenommen.

Die neuen Verbindungen können in Form von Säureadditionssalzen, insbesondere pharmazeutisch verwendbaren, nicht-toxischen Salzen, z.B. mit organischen Säuren, wie Chlorwasserstoff-, Bromwasserstoff-, Schwefel- oder Phosphorsäure, oder mit organischen, wie aliphatischen, cycloaliphatischen, cycloaliphatisch-aliphatischen, aromatischen, araliphatischen, heterocyclischen oder heterocyclisch-aliphatischen Carbon- oder Sulfonsäure, z.B. Essig-, Propion-, Bernstein-, Glykol-, Milch-, Aepfel-, Wein-, Zitronen-, Ascorbin-, Malein-, Phenylessig-, Benzoe-, 4-Aminobenzoe-, Anthranil-, 4-Hydroxybenzoe-, Salicyl-, Aminosalicyl-, Embon- oder Nicotin-, sowie Methansulfon-, Aethansulfon-, 2-Hydroxyäthansulfon-, Aethylensulfon-, Benzolsulfon-, p-Toluolsulfon-, Naphthalinsulfon-, Sulfanil- oder Cyclohexylsulfaminsäure, vorliegen. Salze dieser Art können z.B. durch Behandeln der freien Verbindungen, mit den Säuren oder mit geeigneten Anionenaustauscherharzen erhalten werden.

Infolge der engen Beziehungen zwischen den neuen Verbindungen in freier Form und in Form ihrer Salze sind im Vorausgegangenen und nachfolgend unter den freien Verbindungen oder den Salzen sinn- und zweckmässig gegebenenfalls auch die entsprechenden Salze bzw. freien Verbindungen zu verstehen.

Die neuen Verbindungen können als Isomerengemische, wie Racemate oder Diastereoisomerengemische, oder in Form der reinen Isomeren, wie optisch aktiven Komponenten, vorliegen. Die Auftrennung von erhaltenen Isomerengemischen in die reinen Isomeren kann nach den bekannten Methoden geschehen. Racemate lassen sich z.B. auf Grund physikalisch-chemischer Unterschide, wie z.B. solchen der Löslichkeit, ihrer diastereomeren Salze, oder durch fraktioniertes Kristallisieren aus einem optisch aktiven Lösungsmittel, oder durch Chromatographie, insbesondere Dünnschichtchromatographie, an einem optisch aktiven Trägermaterial, in die optisch aktiven Antipoden auftrennen. Dabei isoliert man vorteilhafterweise das pharmakologisch wirksamere oder weniger toxische reine Isomere, insbesondere den wirksameren oder weniger toxischen aktiven Antipoden.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Die Erfindung betrifft auch die als Zwischenprodukte erhältlichen neuen Verbindungen der allgemeinen Formel Va bzw. Vb

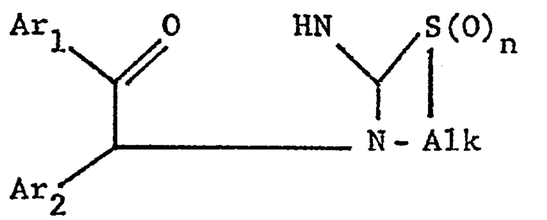

(Va)

bzw.

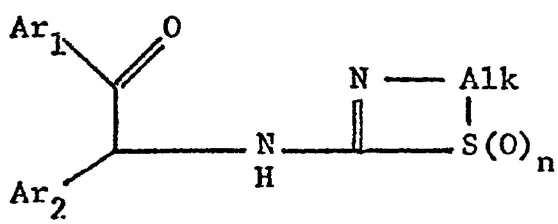

(Vb)

oder ihre Tautomeren, worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, und deren Salze. In den oben erwähnten biologischen Testanordnungen sind sie ungefähr gleich wirksam wie die Dihydroverbindungen der Formel I und können als Antiinflammatorika, z.B. zur Behandlung von rheumatischer Arthritis, verwendet werden.

Die neuen Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen

oder organischen, festen oder flüssigen, pharmazeutisch verwendbaren Trägerstoffen enthalten, die sich zur enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können,

werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Die Einzeldosis für einen Warmblüter von etwa 70 kg Gewicht beträgt zwischen 0,1 und 0,75 g, die Tagesdosis zwischen 0,2 und 1,0 g.

Die folgenden Beispiele dienen zur Illustration der Erfindung; Temperaturen werden in Celsiusgraden angegeben.

Beispiel 1

Eine Mischung aus 5 g α-Brom-desoxyanisoin, 3 g 2-Aminothiazolin und 30 ml Aethanol wird 4 Stunden bei 60°, danach 2 Stunden am Rückfluss und anschliessend noch 12 Stunden bei Raumtemperatur gerührt. Die dabei ausgefallenen Kristalle werden abgenutscht und mit Aethanol und Diäthyläther nachgewaschen. Man erhält so rohes 5,6-Di-(p-methoxy-phenyl)-imidazo[2,1-b]dihydro-thiazol. F. nach dem Umkristallisieren aus Toluol-Petroläther 152-154°

Beispiel 2

Eine Suspension von 7 ml 1,2-Dibromäthan, 7 g Natriumcarbonat und 55 ml Isopropanol wird bei Raumtemperatur gerührt und innert einer Stunde mit der Suspension von 4,7 g 4,5-Diphenyl-imidazolidin-2-thion in 110 ml 1,5%-iger Natronlauge versetzt. Das Reaktionsgemisch wird 7 Stunden am Rückfluss gekocht, dann werden das Isopropanol und das Dibromäthan am Rotationsverdampfer entfernt und die verbleibende Suspension wird mit Toluol extrahiert. Der Toluolextrakt wird mit Sole gewaschen, über Natriumsulfat getrocknet und eingedampft. Der Rückstand wird auf Silicagel chromatographiert. Nach dem Abtrennen unpolarer Verunreinigungen mit Aethylacetat wird das cis-5,6-Diphenyl-2,3,5,6-tetrahydro-imidazo[2,1-b]thiazol mit einem Gemisch aus Aethylacetat:Methanol = 99:1 als farbloses Oel eluiert. Es kristallisiert spontan zu weissen Kristallen vom Smp. 110-113° (Sinterpunkt 103°)

Beispiel 3

Eine Suspension von 3,5 ml 1,2-Dibromäthan, 3,5 g Natriumcarbonat und 30 ml Isopropanol wird unter Rühren bei Raumtemperatur während einer Stunde mit der Suspension von 3 g 4,5-Di-anisyl-2-mercapto-imidazol in 50 ml 1,5%-iger Natronlauge versetzt. Das Reaktionsgemisch wird 6 Stunden am Rückfluss gekocht, dann werden das Isopropanol und das Dibromäthan am Rotationsverdampfer entfernt und die verbleibende Suspension wird mit 10 ml 20%-iger Kalilauge versetzt und mit Aethylacetat extrahiert. Die organischen Phasen werden mit Sole gewaschen, mit Natriumsulfat getrocknet und eingedampft. Aus dem Rückstand erhält man nach dem Umkristallisieren aus Toluol-Petroläther das 5,6-Di-(p-methoxy-phenyl)-imidazo[2,1-b]dihydro-thiazol vom F. 152-154°.

Beispiel 4

14,8 g 4,5-Di-(p-Methoxy-phenyl)-2-ß-hydroxyäthylmercapto-imidazol werden in 50 ml absolutem Pyridin gelöst und unter Rühren bei ca -5° Innentemperatur zu einer Lösung von 15,8 g Benzolsulfochlorid in 60 ml absolutem Pyridin getropft. Anschliessend rührt man 60 Stunden bei 0° aus. Das Reaktionsgemisch wird auf Eiswasser gegossen und mit Dichlormethan extrahiert. Die mit Wasser 3-mal gewaschenen und vereinigten Dichlormethanextrakte werden über Natriumsulfat getrocknet und zur Trockne eingedampft. Das viskose Oel wird aus Toluol-Petroläther kristallisiert. Das so erhaltene 5,6-Di-(p-methoxy-phenyl)-imidazo[2,1-b]dihydro-thiazol vom F. 152-154°.

Das Ausgangsmaterial kann z.B. hergestellt werden:

2,5 g Natrium werden in 220 ml Aethanol gelöst. Dazu gibt man 35 g 4,5-Dianisyl-imidazolin-2-thion. Man erhält eine Suspension. Innerhalb 2 Minuten werden dazu bei Raumtemperatur (Rühren) 15,7 ml 2-Chloräthanol getropft. Man rührt 1 Stunde bei 60° und 4 Stunden am Rückfluss weiter. Anschliessend wird die schwache Suspension zur Trockne eingedampft. Der Rückstand wird aus Aceton/Wasser gereinigt.

Beispiel 5

Ein Gemisch aus 60 g N-Aethyl-diisopropylamin, 70 g 2-Aminodihydrothiazol, 150 g α-Brom-desoxyanisoin und 800 ml Acetonitril wird 48 Stunden bei Raumtemperatur gerührt. Das dabei entstandene 5,6-Di-(p-methoxyphenyl)-imidazo[2,1-b]dihydro-thiazolin wird abgenutscht und mit Acetonitril nachgewaschen. Durch Eindampfen des Filtrates und Verteilen des so vom Lösungsmittel befreiten Rückstandes zwischen Aethylacetat und Wasser erhält man weitere 50 g Rohprodukt. Durch Umkristallisieren des Rohproduktes aus Toluol erhält man 5,6-Di-(p-methoxyphenyl)-imidazo[2,1-b]dihydro-thiazol vom F. 152-154°.

Beispiel 6

Zu einer Lösung von 23 g p-Toluolsulfochlorid in 80 ml Pyridin wird bei 0° unter Rühren die Lösung von 20 g 2-(2-Hydroxy-äthyl-thio)-4(5)-3'-pyridyl-5(4)-phenyl-imidazol zugegeben. Das Reaktionsgemisch wird 16 Stunden bei 0° gerührt, auf Eis-Wasser gegossen und mit Aether extrahiert. Aus den Aetherextrakten erhält man nach dem Waschen mit Wasser und Trocknen über Natriumsulfat einen Eindampfrückstand der nach dem Chromatographieren auf Silicagel mit Toluol und Essigester und nach dem Umkristallisieren der entsprechenden Fraktionen aus Essigester das Isomerengemisch 5(6)-(3-Pyridyl)-6(5)-phenyl-imidazo-[2,1-b]-dihydro-thiazol als weisse Kristalle vom F. 150-151° ergibt.

Das Ausgangsmaterial lässt sich wie folgt erhalten:

10,8 g Benzyl-pyridyl(3)-keton werden zusammen mit 40 ml Pyridin und einer Lösung von 8 g Hydroxylaminhydorchlorid in 15 ml Pyridin während 6 Stunden bei 100° gerührt. Das Reaktionsgemisch wird auf Eis/Wasser gegossen und 15 Minuten weitergerührt. Die ausgefallenen Kristalle werden abgenutscht, mit Wasser gewaschen und am Hochvakuum getrocknet. Man erhält das Benzyl-pyridyl(3)-keton-oxim vom F. 122-126°.

Zu einer bei -10° gerührten Lösung von 8,5 g Benzyl-pyridyl(3)-keton-oxim in 20 ml Pyridin wird innert 5 Minuten die Lösung von 7,7 g p-Toluolsulfochlorid in 15 ml Pyridin zugetropft. Das Reaktionsgemisch wird 24 Stunden im Eisschrank aufbewahrt und dann auf Eis/Wasser gegossen. Nach längerem Rühren und Verreiben erstarrt das ausgefallene Oel zu Kristallen. Diese werden abgenutscht, mit Wasser gewaschen und am Hochvakuum getrocknet. Man erhält 11,6 g eines Rohproduktes vom F. 87-92°, welches nach Dünnschichtchromatographie jedoch noch Edukt enthält. Es wird direkt in der nächsten Stufe eingesetzt.

11,6 g Rohprodukt (Benzyl-pyridyl(3)-keton-oxim-p-toluolsulfoester) werden in 90 ml absolutem Aethanol suspendiert und bei 0° unter Rühren die Lösung von 3,7 g Kalium-tert.-butylat in 30 ml absolutem Aethanol zugetropft. Das Reaktionsgemisch wird 2 Stunden bei 0° gerührt. Die Suspension wird abgenutscht und das Filtrat sofort in der nächsten Stufe eingesetzt.

3,6 g Natriumthiocyanat werden in 60 ml Aethanol gelöst und mit 4,5 ml konzentrierter Salzsäure versetzt.

Die Suspension wird abgenutscht und das Filtrat wird zusammen mit der alkoholischen Lösung des erhaltenen α-Amino-benzyl-pyridyl(3)-keton während 18 Stunden am Rückfluss gehalten. Nach dem Abkühlen lässt sich aus dem Reaktionsgemisch 2,8 g rohes 4-Phenyl-5-pyridyl(3)-2-mercapto-imidazol abnutschen. Das Filtrat enthält weitere Mengen des Produktes. Nach dem Umkristallisieren aus Dimethylformamid-Wasser schmilzt es bei 290-300°.

0,8 g Natrium werden in 200 ml Aethanol gelöst. Die Lösung wird mit 8 g 1-Phenyl-5-pyridyl(3)-2-mercapto-imidazol versetzt und zum Rückfluss erhitzt. Nachdem eine klare Lösung entstanden ist, werden 2,3 ml 2-Chloräthanol zugetropft und das Reaktionsgemisch wird 16 Stunden am Rückfluss gekocht. Das Lösungsmittel wird am Vakuum abgedampft und der Rückstand zwischen Wasser und Essigester verteilt. Die organischen Phasen werden mit Wasser und Sole gewaschen, mit Natriumsulfat getrocknet und eingedampft. Als Rückstand erhält man kristallines 2-(2-Hydroxy-äthyl-thio)-4(5)-3'-pyridyl-5(4)-phenyl-imidazol. Es schmilzt nach dem Umkristallisieren aus Essigester/Aethanol bei 157-159°C.

In analoger Weise, ausgehend von den entsprechenden Ausgangsstoffe lassen sich die folgenden Verbindungen herstellen:

das 5,6-Di-(p-methyl-phenyl)-2,3,5,6-tetrahydro-imidazo[2,1-b]thiazol,

das 5,6-Di-(p-chlor-phenyl)-2,3,5,6-tetrahydro-imidazo[2,1-b]thiazol,

das 5,6-Di-(m-chlor-phenyl)-2,3,5,6-tetrahydro-imidazo[2,1-b]thiazol,

das 5,6-Di-(p-methoxy-phenyl)-3-methyl-2,3,5,6-tetrahydro-imidazo[2,1-b]thiazol oder

das 6,7-Di-(p-methoxy-phenyl)-2,3,4,5,6,7-hexa-hydro-imidazo[2,1-b]thiazin.

Beispiel 7

14,3 g 2-[N-(1,2-Bis-p-methoxy-phenyl-2-hydroxy-äthyl]-amino-thiazolin werden in 24,3 ml konzentrierter Schwefelsäure gelöst und eine Stunde bei Raumtemperatur gerührt. Das Reaktionsgemisch wird auf Eis gegossen, mit 2-n Sodalösung alkalisch gestellt und mit Essigester extrahiert Die organischen Phasen werden mit Wasser gewaschen, über Natriumsulfat getrocknet und eingedampft. Aus dem Rückstand erhält man durch Umkristallisieren aus Toluol-Petroläther das 5,6-trans-Di-(p-methoxyphenyl)-2,3,5,6-tetrahydro-4H-imidazol[2,1-b]thiazol vom F. 125-126°.

Das verwendete Ausgangsmaterial lässt sich z.B. wie folgt erhalten:

45 g Brom-desoxy-anisoin werden in 170 ml Acetonitril suspendiert. Die Suspension wird mit 23 ml N-Aethyldiisopropylamin und mit 15,4 g 2-Aminothiazolin versetzt, 2 Stunden bei Raumtemperatur gerührt, abgenutscht und das Nutschgut mit wenig Essigester nachgewaschen. Man erhält so das 2-[N-(1,2-Bis-p-methoxyphenyl-2-oxo-äthyl)-amino-thiazolin bzw. das 2-Imino-3-(1,2-bis-p-methoxy-phenyl-2-oxo-äthyl)-thiazolin vom F. = 95-98°.

In analoger Weise erhält man

das 2-[N-(1,2-Bis-p-methoxyphenyl-2-oxo-äthyl)-amino-5-methyl-thiazolin bzw. 2-Imino-5-methyl-3-(1,2-Bis-p-methoxyphenyl-2-oxo-äthyl)-thiazolin oder deren Tautomeren,

das 2-[N-(1,2-Bis-p-methoxyphenyl-2-oxo-äthyl)-amino-4-methyl-thiazolin bzw: 2-Imino-4-methyl-3-(1,2-bis-p-methoxyphenyl-2-oxo-äthyl)-thiazolin oder deren Tautomeren,

das 2-[N-(1,2-Bis-phenyl-2-oxo-äthyl)-amino-thiazolin bzw. 2-Imino-3-(1,2-bis-phenyl-2-oxo-äthyl)-thiazolin oder deren Tautomeren vom F. 121-123°, oder

das 2-[N-(1,2-Bis-p-methoxyphenyl-2-oxo-äthyl)-amino-5,6-dihydro-4H-thiazin bzw. 2-Imino-3-(1,2-bis-p-methoxyphenyl-2-oxo-äthyl)-5,6-dihydro-4H-thiazin oder deren Tautomeren.

31,5 g 2-[N-(1,2-Bis-p-methoxyphenyl-2-oxo-äthyl)-amino-thiazolin oder dessen Isomeren werden in 350 ml Methanol gelöst und portionsweise mit 4,26 g Natriumborhydrid versetzt. Die Suspension wird 2 Stunden bei Raumtemperatur gerührt, dann abgenutscht und mit Methanol nachgewaschen. Man erhält so das 2-[N-(1,2-Bis-p-methoxyphenyl-2-hydroxy-äthyl)-amino-thiazolin vom F. 185-187°.

Ausgehend von den entsprechenden Thiazolinverbindungen lassen sich in analoger Weise die folgenden Verbindungen erhalten:

das 5-Phenyl-6-p-chlorphenyl-2,3,5,6-tetrahydro-imidazo[2,1-b]thiazol,

das 6-Phenyl-5-p-chlorphenyl-2,3,5,6-tetrahydro-imidazo[2,1-b]thiazol,

das 5,6-Di-(p-methoxyphenyl)-2-methyl-2,3,5,6-tetrahydro-imidazo[2,1-b]thiazol oder

das 5,6-Di-(p-methoxyphenyl-3-methyl-2,3,5,6-tetrahydro-imidazo[2,1-b]thiazol.

Beispiel 8

2 g 5,6-Di-p-methoxyphenyl-2,3-dihydro-4H-imidazo[2,1-b]thiazol werden in 33 ml Aethanol suspendiert und innert 5 Minuten mit 7,91 ml 30%-igem Wasserstoffperoxyd versetzt. Das Reaktionsgemisch wird 90 Minuten am Rückfluss gehalten, abgekühlt, auf 200 ml Eiswasser gegossen und mit Essigester extrahiert. Aus dem Eindampfrückstand

der mit Wasser gewaschenen und über Natriumsulfat getrockneten organischen Phasen erhält man durch Umkristallisieren das 5,6-Di-p-methoxyphenyl-imidazo[2,1-b]2,3-dihydrothiazol-sulfoxid vom F. 176-177°.

Beispiel 9

0,69 g Natrium werden in 60 ml Aethanol gelöst und mit 9,36 g 2-Mercapto-4,5-di-p-methoxyphenyl-imidazol versetzt. Das Reaktionsgemisch wird 30 Minuten gerührt, mit 3,1 g 3-Chlor-1-propanol versetzt, 2 Stunden am Rückfluss gehalten, abgekühlt, klar-filtriert und zur Trockne eingedampft. Der Rückstand, das rohe 2-(3-Hydroxy-propylthio)-4,5-di-p-methoxyphenyl-imidazol wird in 50 ml Thionylchlorid 30 Minuten am Rückfluss gehalten. Das überschüssige Thionylchlorid wird abgedampft, durch Abdestillieren von 100 ml noch zugesetztem Chloroform abgetrieben und der Rückstand zusammen mit 100 ml Aethanol und 50 ml 40%-iger Kalilauge 4 Stunden am Rückfluss gehalten. Danach wird das Reaktionsgemisch am Rotationsverdampfer eingedampft, mit Eiswasser versetzt und mit Essigester extrahiert. Die organischen Phasen werden mit Wasser gewaschen, getrocknet und eingedampft. Der Rückstand wird mit Toluol:Essigester 1:1 auf Silicagel chromatrographiert. Durch Umkristallisieren der entsprechenden Fraktionen aus Aceton erhält man das 6,7-Di-p-methoxyphenyl-2,3,4,5-tetrahydro-imidazo[2,1-b] (1,3)-thiazin vom 189-191°.

Beispiel 10

Analog Beispiel 9 wird aus dem 2-Mercapto-4,5-di-p-chlorphenyl-imidazol mit 2-Chloräthanol das 2-(2-Hydroxy-äthyl-thio)-4,5-di-p-chlorphenyl-imidazol vom F. 197-199° hergestellt und daraus durch Cyclisation mit Thionylchlorid das 5,6-Di-p-chlorphenyl-imidazo[2,1-b]dihydro-thiazol vom F. 199-204°.

Analog kann auch das 5,6-di-p-Methoxyphenyl-4H-imidazo[2,1-b]dihydro-thiazol hergestellt werden.

Beispiel 11

0,5 g 5,6-Di-p-Methoxyphenyl-2,3-dihydro-4H-imidazo[2,1-b]thiazol werden in 1,5 ml Eisessig suspendiert und mit 0,9 ml 30%-igem Wasserstoffperoxid versetzt. Das Reaktionsgemisch wird 7 Stunden bei 60° gerührt, dann auf Eis-Wasser gegossen und mit Essigester extrahiert. Die organischen Phasen werden mit 1-n Natronlauge gewaschen bis die Extrakte basisch bleiben, dann mit Wasser und Sole neutralgewaschen, über Natriumsulfat getrocknet und eingedampft. Der Rückstand wird aus Essigester kristallisiert, und aus Essigester-Petroläther umkristallisiert. Man erhält so das 5,6-di-p-Methoxyphenyl-imidazo[2,1-b]dihydro-thiazol-sulfon vom F. 186-187°.

Beispiel 12

35 g 2-[N-(1,2-Bis-p-methoxyphenyl-2-oxo-äthyl)-aminothiazolin (vgl. Beispiel 7) werden zusammen mit 250 ml Isopropanol und 1 ml Bromwasserstoff (48%-ig) 2 Stunden am Rückfluss gehalten. Durch Abkühlen im Eisbad und Abnutschen des Produktes erhält man das 5,6-Di-(p-methoxyphenyl)-imidazol[2,1-b]dihydro-thiazol vom F. 155-156°.

Aus der Mutterlauge lässt sich das Hydrobromid des 5,6-Di-(p-methoxyphenyl)-imidazo[2,1-b]dihydro-thiazols gewinnen, Smp. 200-210°.

Beispiel 13

Zu einer Suspension von 11,8 g 5,6-di-p-Anisyl-2,3,5,6-tetrahydro-4H-imidazo[2,1-b]-thiazol in 30 ml Wasser werden 17 ml 1-n Salzsäure und 5 g N-Benzolsulfo-

nyl-L(+)-glutaminsäure zugegeben. Das Gemisch wird durch Erwärmen gelöst und durch Abkühlen zur Kristallisation gebracht. Das L-5,6-di-p-Anisyl-2,3,5,6-tetrahydro-4H-imidazo[2,1-b]thiazol N-benzol-sulfonyl L(+)-glutamat wird abgenutscht, mit wenig Wasser gewaschen, in Wasser suspendiert und mit verdünnter Natronlauge zerlegt. Daraus wird das L-5,6-di-p-Anisyl-2,3,5,6-tetrahydro-4H-imidazo[2,1-b]thiazol mit Aethylacetat extrahiert.

Beispiel 14

Pharmazeutische Präparate enthaltend

| | |
|---|---|
| 5,6-Di-(p-methoxy-phenyl)-imidazo[2,1-b] dihydro-thiazol | 50 mg |
| Magnesiumstearat | 5 mg |
| Lactose | 100 mg |

Diese Bestandteile werden gesiebt, gut gemischt und die Mischung in Hargelatinekapseln gefüllt.

Beispiel 15

Tabletten enthaltend

| | |
|---|---|
| 5,6-Di-(p-methoxy-phenyl)-imidazo[2,1-b] dihydro-thiazol | 100 mg |
| Calzium-dihydrat | 150 mg |
| Saccharose | 20 mg |
| Stärke | 10 mg |
| Talkum | 5 mg |
| Stearinsäure | 3 mg |

Die Saccharose, das Calzium-dihydrat und der Wirkstoff werden gemischt und mit einer 10%-ige Gelatinelösung granuliert. Das feuchte Granulat wird gesiebt, getrocknet, mit der Stärke, dem Talkum und der Stearinsäure vermischt, gesiebt und zu Tabletten verpresst.

Patentansprüche

1. 1,3-Diaza-cyclopent-2-eno[2,1-b](1-thia-3-aza-cycloalkane) der allgemeinen Formel I

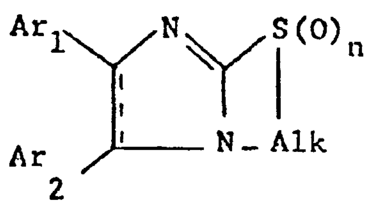

(I)

deren 1,3-Diaza-cyclopent-2-en-ring eine weitere Doppelbindung aufweisen kann, Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschieden ist, wenn Alk Aethylen und der 1,3-Diaza-cyclopent-2-en-ring einen Imidazolring darstellt, und deren Salze.

2. Verbindungen der Formel I, worin $Ar_1$ und $Ar_2$ unabhängig voneinander einen gegebenfalls durch Niederalkyl, Niederalkoxy, Halogen oder Trifluormethyl substituierter Phenylrest, einen Pyridyl oder Thienylrest darstellen, Alk ein Niederalkylenrest, der das Schwefel- und Stickstoffatom über 2-3 Kohlenstoffatome miteinander verbindet und n 0, 1 oder 2 bedeutet und ihre Salze.

3. Verbindungen der Formel II

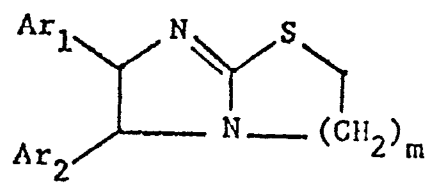

(II)

worin $Ar_1$ und $Ar_2$ unabhängig voneinander einen gegebenenfalls durch Niederalkoxy, wie Methoxy oder Halogen, insbesondere Chlor, substituierten Phenylrest bedeuten und m in erster Linie 1, ferner auch 2 ist, und ihre Salze.

4. 5,6-Diphenyl-2,3,5,6-tetrahydroimidazo[2,1-b]thiazol und dessen Salze.

5,6-Di-(p-methyl-phenyl)-2,3,5,6-tetrahydro-imidazo[2,1-b]thiazol und dessen Salze.

5,6-Di-(p-chlor-phenyl)-2,3,5,6-tetrahydro-imidazo[2,1-b]thiazol und dessen Salze.

5,6-Di-(m-chlor-phenyl)-2,3,5,6-tetrahydro-imidazo[2,1-b]thiazol und dessen Salze.

5,6-Di-(p-methoxy-phenyl)-2- oder 3-methyl-2,3,5,6-tetrahydro-imidazo[2,1-b]thiazol und dessen Salze.

5,6-Di-(p-methoxy-phenyl)-3-methyl-2,3,5,6-tetrahydro-imidazo[2,1-b]thiazol und dessen Salze.

5. 5,6-Di-(p-methoxy-phenyl)-2,3,4,5,6,7-hexahydro-imidazol[2,1-b]thiazin und dessen Salze.

6. Verbindungen der Formel III

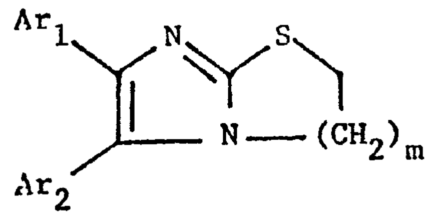

(III)

worin $Ar_1$ und $Ar_2$ unabhängig voneinander einen gegebenenfalls durch Niederalkoxy, wie Methoxy oder Halogen, insbesondere Chlor, substituierten Phenylrest bedeuten und m in erster Linie 1, ferner auch 2 ist, und ihre Salze.

7. 5,6-Di-(p-methoxy-phenyl)-imidazo[2,1-b]dihydrothiazol und dessen Salze.

8. 5(6)-(3-Pyridyl)-6(5)-phenyl-imidazol[2,1-b] dihydro-thiazol und dessen Salze.

9. Verfahren zur Herstellung von 1,3-Diaza-cyclopent-2-eno[2,1-b](1-thia-3-aza-cycloalkane) der allgemeinen Formel I

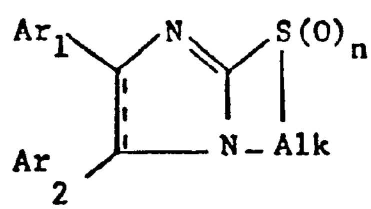

(I)

deren 1,3-Diaza-cyclopent-2-en-ring eine weitere Doppelbindung aufweisen kann. Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschie-

den ist, wenn Alk Aethylen und der 1,3-Diaza-cyclopent-2-en-ring einen Imidazolring darstellt, und deren Salze. dadurch gekennzeichnet, dass man Verbindungen der Formel IV

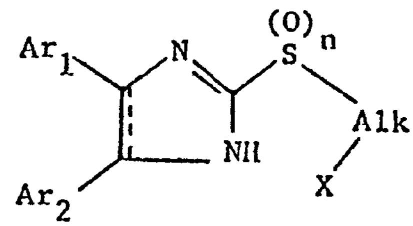

(IV)

worin X eine reaktionsfähige veresterte Hydroxygruppe darstellt, ringschliesst, oder dass man Verbindungen der Formel Va bzw. Vb

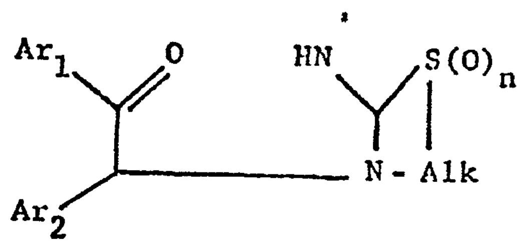

(Va)

bzw.

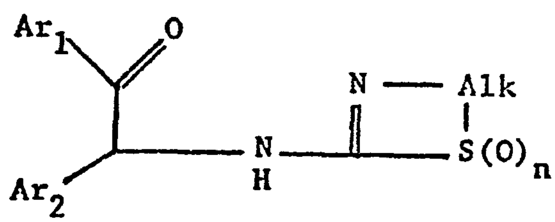

(Vb)

ringschliesst, und, wenn erwünscht, in gegebenenfalls erhaltenen Verbindungen, worin n 0 ist, das Thiaatom zur Sulfinyl- oder Sulfonylgruppe oxidiert, und/oder, wenn erwünscht, erhaltene freie Verbindungen in ihre Salze überführt oder erhaltene Salze in die freien Verbindungen umwandelt und/oder ein verfahrensgemäss erhaltenes Isomerengemisch in die einzelnen Isomeren auftrennt.

10. Verbindungen der Formel Va bzw. Vb

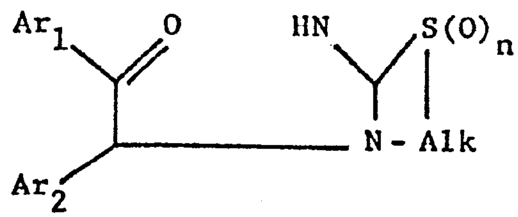

(Va)

bzw.

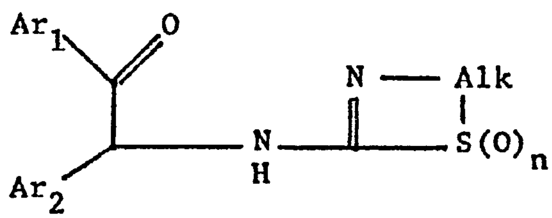

(Vb)

oder ihre Tautomeren, worin Alk Niederalkylen darstellt, welches das Thia- vom Azaatom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist und deren Salze.

11. Verbindungen der in Anspruch 10 gezeigten Formel Va bzw. Vb oder ihre Tautomeren, worin Alk ein Niederalkylenrest ist, der das Schwefel- und Stickstoffatom über 2-3 Kohlenstoffatome miteinander verbindet, $Ar_1$ und $Ar_2$ unabhängig voneinander einen gegebenenfalls durch Niederalkoxy oder Halogen substituierten Phenylrest und n 0, 1 oder 2 bedeutet, und deren Salze.

12. 2-[N-(1,2-Bis-p-methoxyphenyl-2-oxo-äthyl)-amino-thiazolin bzw. 2-Imino-3-(1,2-bis-p-methoxy-phenyl-2-oxo-äthyl)-thiazolin oder deren Tautomere.

2-[N-(1,2-Bis-phenyl-2-oxo-äthyl)-amino-thiazolin bzw. 2-Imino-3-(1,2-bis-phenyl-2-oxo-äthyl)-thiazolin oderen deren Tautomere.

2-[N-(1,2-Bis-p-methoxyphenyl-2-oxo-äthyl)-amino-5,6-dihydro-4H-thiazin bzw. 2-Imino-3-(1,2-bis-p-methoxyphenyl-2-oxo-äthyl)-5,6-dihydro-4H-thiazin oder deren Tautomere.

13. Pharmazeutische Präparate enthaltend eine der in den Ansprüchen 1-8 und 10-12 genannten Verbindungen.

14. Verwendung einer der in den Ansprüchen 1-8 und 10-12 beanspruchten Verbindungen oder eines ihrer therapeutisch verwendbaren Salze zur Herstellung eines Arzneimittels auf nicht-chemischem Wege.

15. Eine der in den Ansprüche 1-8 und 10-12 beanspruchten Verbindungen oder eines ihrer therapeutisch verwendbaren Salze als Arzneimittel.